(19) Europäisches Patentamt
European Patent Office
Office européen des brevets

(11) EP 1 231 199 B1

(12) EUROPÄISCHE PATENTSCHRIFT

(45) Veröffentlichungstag und Bekanntmachung des Hinweises auf die Patenterteilung:
**20.07.2005 Patentblatt 2005/29**

(51) Int Cl.[7]: **C07C 45/74**, C07C 49/203

(21) Anmeldenummer: **02000633.4**

(22) Anmeldetag: **11.01.2002**

(54) **Verfahren zur Kondensation von Aldehyden mit Ketonen mittels Mehrphasenreaktion**

Process for the condensation of aldehydes with ketones by multiphase reaction

Procédé pour la condensation d'aldéhydes avec des cétones par réaction multiphasique

(84) Benannte Vertragsstaaten:
**AT BE CH CY DE DK ES FI FR GB GR IE IT LI LU MC NL PT SE TR**

(30) Priorität: **10.02.2001 DE 10106186**

(43) Veröffentlichungstag der Anmeldung:
**14.08.2002 Patentblatt 2002/33**

(73) Patentinhaber: **Oxeno Olefinchemie GmbH
45772 Marl (DE)**

(72) Erfinder:
- **Protzmann, Guido, Dr.
64673 Zwingenberg (DE)**
- **Wiese, Klaus-Diether, Dr.
45721 Haltern (DE)**
- **Büschken, Wilfried, Dr.
45721 Haltern (DE)**

(56) Entgegenhaltungen:
**EP-A- 1 057 524** **EP-A- 1 057 525**
**EP-A- 1 106 596**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 231 199 B1

Printed by Jouve, 75001 PARIS (FR)

## Beschreibung

Die vorliegende Erfindung betrifft ein Verfahren zur Durchführung von Mehrphasenreaktionen, insbesondere die Herstellung von α,β-ungesättigten Ketonen durch Kondensation von Aldehyden mit Ketonen.

α,β-ungesättigte Ketone werden aufgrund ihrer Reaktivität für eine Vielzahl organischer Synthesen genutzt. Sie sind Zwischenprodukte für die Herstellung von Riechstoffen und Pharmazeutika.

Es ist aus Houben-Weyl, Methoden der organischen Chemie, Band 7/1, Seiten 77 ff, und Organic Reactions, Band 16, Seiten 27- 47, 69-78, 177 ff, bekannt, dass man Aldehyde mit Ketonen zu α,β-ungesättigten Ketonen umsetzen kann. Temperaturen von 5 bis 25°C sind für diese Kondensationen bevorzugt (Organic Reactions, loc. cit., Seite 77). Die bei diesen Verfahren angewandten, zahlreichen Katalysatoren, z. B. Alkali- und Erdalkalihydroxide, organische Basen, Alkalisalze, Alkoholate, begünstigen zugleich auch die Eigenkondensation der Aldehyde bzw. Ketone und lassen daher auch Nebenprodukte entstehen. Die Aufarbeitung solcher Gemische ist mit größerem Aufwand verbunden, da der verwendete Katalysator wieder entfernt oder neutralisiert werden muss. Die Ausbeuten an Zielprodukten sind oft unbefriedigend.

In DE 2150992 wird ein Verfahren zur Herstellung von α,β-ungesättigten Ketonen aus Aldehyden und Ketonen beschrieben. Dabei wird die Aldolkondensation durch einen Kontakt, der im Wesentlichen aus Zinkoxid besteht, katalysiert. Die Umsetzung erfolgt bei Temperaturen von 140°C bis 200°C. Das Keton wird im Überschuss eingesetzt. Das Reaktionsgemisch wird destillativ aufgearbeitet. Bei molaren Verhältnissen zwischen Keton und Aldehyd von 1,3/1 bis 5,7/1 werden bei Umsätzen von 66 % bis 82 %, bezogen auf den im Unterschuss eingesetzten Aldehyd, α,β-ungesättigten Ketone abhängig von der Art der Einsatzstoffe in Selektivitäten von 75 % bis 93 % erhalten. Da die Reaktionsgemische nur zu 20 % bis 60 % aus dem Zielprodukt bestehen, ist der Trennaufwand beträchtlich.
EP 0 792 862 A1 offenbart ein Verfahren zur Umsetzung von Aldehyden mit Ketonen an einem komplexen Magnesium-Aluminium-Hydroxid. Der im Unterschuss eingesetzte Aldehyd bildet dabei mit dem Einsatzketon sowohl das Aldoladditions- als auch das Aldolkondensationsprodukt im molaren Verhältnis von 0,7/1 bis 1,40/1. Bei einem Aldehyd-Umsatz von 96 % bis 98 % entstehen diese beide Stoffe zusammen in einer Selektivität, bezogen auf Aldehyd, von nur 71 % bis 79 %.

US 5 583 263 beschreibt ein zweistufiges Verfahren zur Herstellung von α,β-ungesättigten Ketonen, insbesondere die Umsetzung von n-Butyraldehyd mit Aceton: In der ersten Stufe wird das Eduktgemisch, das Aceton im Überschuss enthält, an einem basischen Ionenaustauscherharz zu β-Hydroxyketonen umgesetzt. n-Butyraldehyd wird bei vollständigem Umsatz in einer Selektivität bis zu 88 % zu 4-Hydroxyheptanon umgesetzt. Daneben entsteht aus Aceton in bis zu 95 %-iger Selektivität Diacetonalkohol. Das Rohgemisch der ersten Stufe wird in der zweiten Stufe sauer katalysiert zu den α,β-ungesättigten Ketonen dehydratisiert. Als Katalysator werden starke Säuren oder ein stark saueres Ionenaustauscherharz eingesetzt. Die Ausbeute an Hept-3-en-2-on, dem Kondensationprodukt aus n-Butyraldehyd und Aceton, liegt bezogen auf n-Butyraldehyd bei 85 %. Aus dem Diaceton entsteht Mesityloxid. Dieses Verfahren ist demnach ein Koppelprozess für die Herstellung von zwei α,β-ungesättigten Ketonen. Die Anwendung dieses Verfahren ist nachteilig, wenn nur ein Aldolkondensationsprodukt, insbesondere das aus Aldehyd und Keton, das Zielprodukt ist.

Ein weiteres zweistufiges Verfahren zur Herstellung von α,β-ungesättigten Ketonen aus Aldehyden und Aceton ist in WO 91/07368 offengelegt. In der ersten Stufe wird der Aldehyd mit im Überschuss eingesetzten Aceton unter der Katalyse von cyclischen Aminen (Perhydroisoindol- und Pyrrolidinderivate) in Gegenwart von Wasser zu den entsprechenden β-Hydroxyketon umgesetzt. Nach erfolgter Aldoladdition wird mit wässriger Mineralsäure (Schwefelsäure, Salzsäure) ein pH-Wert von 4,5 eingestellt und ein Gemisch aus Aceton und Wasser abdestilliert. Das Rohgemisch wird unter Wasserabspaltung zum Zielprodukt umgesetzt, indem es mit einem Gemisch aus Salzsäure und Chloroform versetzt und unter Rückfluss gekocht wird. Die Isolierung des Zielproduktes geschieht durch Phasentrennung, Waschen und Destillation. Die Ausbeuten an α,β-ungesättigten Methylketonen bezogen auf eingesetzten Aldehyd liegen zwischen 80 % und 90 %. Nachteilig bei diesem Verfahren sind vor allen der hohe Aufarbeitungsaufwand und der Einsatz und Verbrauch an Hilfsstoffen.

Nach EP 0 816 321 A1 werden α,β-ungesättigte Methylketone diskontinuierlich durch gekreuzte Aldolkondensation von Aldehyden mit Aceton hergestellt. Als Katalysator wird 2%-ige Natronlauge verwendet und Aceton im Überschuss eingesetzt. Die Umsetzung erfolgt im Temperaturbereich 70-72°C. die Reaktionszeit beträgt ca. 4,5 h. Bei der Umsetzung von Isovaleraldehyd mit Aceton werden beispielsweise 6-Methyl-3-hept-2-on in 66 %-iger und 6-Methyl-4-hydroxyheptan-2-on in 3,3%-iger Ausbeute bezogen auf Isovaleraldehyd, das zu 98,3 % umgesetzt worden ist, erhalten. Dieses Verfahren hat die Nachteile, dass es diskontinuierlich durchgeführt wird und das die Ausbeute an Zielprodukt nicht zufriedendstellend ist.

Da die oben genannten Verfahren hinsichtlich des Verfahrensaufwands und/oder Raum-Zeit-Ausbeuten nicht überzeugen, bestand die Aufgabe, ein wirtschaftlicheres Verfahren zu entwickeln.

Die erwähnten Verfahren umfassen mindestens eine Mehrphasenreaktion (fest/flüssig oder flüssig/flüssig). Daher bestand der Lösungsansatz darin, die Mehrphasenreaktion, insbesondere die zwischen wenig ineinander mischbaren Flüssigkeiten zu verbessern.

Unter Zweiphasenreaktion werden im Folgenden Reaktionen verstanden, die unter Beteiligung von zwei nicht oder teilweise mischbaren fluiden Phasen ablaufen. Bei der Aldolkondensation von einem Aldehyd und einem Keton liegen zwei Flüssigphasen vor, die nicht mischbar sind oder eine Mischungslücke aufweisen. Zu Beginn der Umsetzung bestehen die beiden Phasen aus Edukt und Katalysatorlösung, nach erfolgter Reaktion aus Produkt und Katalysatorphase.

Bei der Zweiphasenreaktion ist das Problem des Stoffüberganges zu überwinden. Die Edukte müssen in die Katalysatorphase transportiert werden und die Produkte daraus zurück. Da die Transportvorgänge häufig langsamer sind als die eigentliche Reaktion, sind solche Reaktionen durch die Geschwindigkeit des Stoffüberganges bestimmt, man spricht von einer transportgehemmten Reaktion.

Um bei der Zweiphasenreaktion, insbesondere bei jenen, bei denen die Phasen kaum ineinander löslich sind, technisch akzeptable Raum-Zeit-Ausbeuten zu erhalten, müssen die Stoffe möglichst innig miteinander in Kontakt gebracht werden. Es muss eine möglichst große Stoffübergangsfläche $a_s$ zwischen den Phasen erzeugt werden. Andererseits müssen die Phasen nach erfolgter Reaktion wieder leicht getrennt werden können. Zu starke Vermischung kann hier zu Schwierigkeiten führen, da Emulsionen entstehen können.

Neben einer hohen Stoffübergangsfläche as sollte in allen Mehrphasenreaktionen ein möglichst hoher Stoffübergangskoeffizient $k_l$ erreicht werden. Insgesamt sollte der sogenannte KLA-Wert, d.h. das Produkt aus $k_l$ und as in der Stoffübergangsgleichung

$$j = k_l * a_s * (C^* - C)$$

mit

- j [Mol/s] der durch die Phasengrenzfläche hindurchtretende Molenstrom der reagierenden Komponente,
- $k_l$ [m/s] Stoffübergangskoeffizient,
- $a_s$ [$m^2$] Phasengrenzfläche im Reaktor,
- C* [Mol/$m^3$] maximale Löslichkeit des Edukts in der zweiten Phase und
- C [Mol/$m^3$] tatsächliche Konzentration des Edukts, die wiederum mit der Reaktionsgeschwindigkeit gekoppelt ist,

maximal sein.

Ein weiteres Problem bei Mehrphasenreaktionen ist die Wärmeabfuhr bei exothermen Reaktionen. Gelingt es, die Reaktionsgeschwindigkeit durch Verbesserung des Stoffüberganges zu erhöhen, muss naturgemäß auch mehr Wärme abgeführt werden, was zu unerwünschter Temperaturerhöhung bis hin zum Durchgehen der Reaktion führen kann.

Daher wird die zweiphasige Aldolkondensation häufig im Rührkessel durchgeführt. Dabei nimmt man die ständige Rückvermischung in Kauf, wodurch die effektive Konzentration der Reaktanden herabgesetzt wird, was zur Absenkung der Raumzeitausbeute führt. Dieser Nachteil muss mit teurem Reaktionsraum bezahlt werden.

Bei der Durchführung einer Zweiphasenreaktion im Strömungsrohr besteht die Gefahr, dass sich die Phasen entmischen und dadurch die Reaktionsgeschwindigkeit stark zurückgeht.

In EP 1 057 524 und EP 1 057 525 werden die Durchführung von Mehrphasenreaktionen, insbesondere Gas-Flüssig-Reaktionen, wie die Hydroformylierung bzw. die Vinylierung, in einem Rohrreaktor beschrieben, wobei ein Belastungsfaktor von größer oder gleich 0,8 verwendet wird.

Im Hinblick auf die voranstehenden Darlegungen besteht ein Bedarf für ein Verfahren, dass die vorstehend genannten Nachteile vermeidet und sich zudem auf einfache Weise technisch realisieren lässt.

Technisch sollte das neue Verfahren folgende Ansprüche an ein Mehrphasenverfahren erfüllen:

- Erzeugung eines hohen und stabilen Stoffübergangs zwischen den beteiligten Phasen
- Einfach ausführbar, möglichst mit üblichen technischen Apparaten
- Einfache und sichere Wärmeabfuhr
- Hohe Betriebssicherheit
- Einfache und sichere Maßstabsübertragung

In Bezug auf die durchzuführende Herstellung von $\alpha,\beta$-ungesättigten Ketonen kommen speziell hinzu:

- Hohe Selektivität, Vermeidung insbesondere hochsiedender Nebenprodukte
- Geringe Bildung von Aldehyd-Aldehyd-Folgeprodukte
- Geringe Bildung von Keton-Keton-Folgeprodukte

- Hohe Raum-Zeit-Ausbeute, kleine Reaktoren

Mit dem erfindungsgemäßen Verfahren wurde ein überraschend einfaches Verfahren zur Durchführung von Zweiphasenreaktionen gefunden, das in einem Rohrreaktor - gegebenenfalls gefüllt mit Füllkörpern oder Einbauten - durchgeführt werden kann und zur Aldolkondensation von einem Aldehyd mit einem Keton zu ungesättigten Ketonen unter hohen Raum-Zeit-Ausbeuten und Selektivitäten geeignet ist.

Gegenstand der vorliegenden Erfindung ist daher ein Verfahren zur Herstellung von α,β-ungesättigten Ketonen der allgemeinen Struktur I

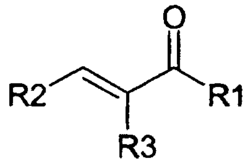

I

in der R1 und R2 für einen verzweigten oder unverzweigten, gesättigten oder ungesättigten aliphatischen oder cycloaliphatisch-aliphatischen Kohlenwasserstoffrest mit 1 bis 20, vorzugsweise 1-16 C-Atomen oder einen gesättigten oder ungesättigten cycloaliphatischen Kohlenwasserstoffrest mit 5 bis 12 C-Atomen, der auch Alkylgruppen als Substituenten und/oder eine Endoalkylengruppe enthalten kann oder aber auch für einen araliphatischen Kohlenwasserstoffrest mit 7 bis 15 C-Atomen, vorzugsweise eine Benzylgruppe, oder einen aromatischen Kohlenwasserstoffrest, vorzugsweise eine Phenylgruppe stehen, R3 Wasserstoff oder einen aliphatischen Kohlenwasserstoffrest mit 1 bis 10 C-Atomen bedeutet, und darüber hinaus R1 und R3 auch zusammen mit den beiden benachbarten Kohlenstoffatomen Glieder eines gemeinsamen alicyclischen Ringes bezeichnen können und weiterhin eine oder mehrere Methylengruppen, die nicht in α-Stellung zu einer Carbonylgruppe stehen, in den Resten R1,R2 und R3 durch ein Sauerstoff- oder Schwefelatom substituiert sein können, durch Umsetzung von einem Aldehyd der allgemeinen Struktur II mit einem Keton der allgemeinen Struktur III, in der R1, R2 und R3 die vorgenannten Bedeutung haben, in flüssiger Phase in einem Rohrreaktor,

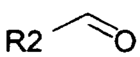

II

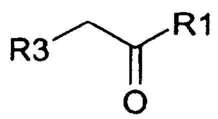

III

dadurch gekennzeichnet, dass der Katalysator in der kontinuierlichen Phase und mindestens ein Edukt in einer dispergierten Phase enthalten ist und der Belastungsfaktor des Rohrreaktors gleich oder größer 0,8 ist.

Die Aldehyde können 1 bis 15 C-Atome, bevorzugt 4 oder 5 C-Atome enthalten. Die Ketone enthalten bevorzugt 3 bis 15 C-Atome, insbesondere wird Aceton eingesetzt.

Die Reste R1, R2 und R3 haben in allen drei Strukturformeln die gleiche Bedeutung.

Der im erfindungsgemäßen Verfahren eingesetzte Rohrreaktor kann Füllkörper oder Einbauten enthalten. Füllkörper im Sinne der vorliegenden Erfindung sind beispielsweise: Raschigringe, Sättel, Pallringe, Telleretten, Maschendrahtringe, Maschendrahtgewebe. Beispiele für Einbauten sind Filterplatten, Strombrecher, Kolonnenböden, Lochbleche oder sonstige Mischvonichtungen. Als Einbauten im Sinne der vorliegenden Erfindung sind aber auch mehrere enge, parallel geschaltete Rohre denkbar, es resultiert also ein Vielrohrreaktor. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, dass der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengrösse mit der Dimension eines längenbezogenen Druckes, definiert als Verhältnis von Massenstrom M [kg/s] aller Komponenten im Reaktor zum Volumenstrom V [$m^3/s$] aller Komponenten unter Betriebsbedingungen, multipliziert mit g = 9,81 $m/s^2$, d. h. PS = (M/V)*g, bedeuten.

Anschaulich wäre PS der statische Druck pro Meter eines Mehrphasengemisches in einem senkrecht stehenden Rohr, wenn alle Phasen mit gleicher Geschwindigkeit strömen würden. PS ist eine reine Rechengrösse, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengrösse, um die Prozessbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte La1 bis Lgb7, sowie im Standardwerk Heinz Brauer, Grundlagen der Einphasen- und Mehrphasenströmungen, Verlag Sauerländer, Aarau und Frankfurt am Main, 1971, nachgeschlagen werden.

Der Druckverlust PD ist bei der Einphasenströmung durch ein leeres Rohr durch

$$PD = Cw*\rho/2*w^2/D$$

mit

- $\rho$ [$kg/m^3$] Dichte des strömenden Mediums unter Betriebsbedingungen,
- w [m/s] Strömungsgeschwindigkeit (Volumenstrom/Querschnittsfläche),
- D [m] Rohrdurchmesser und
- Cw [-] Widerstandsbeiwert des durchströmten Rohres

gegeben.

Bei einer Strömung durch Füllkörper, Schüttungen oder Einbauten ist die Geschwindigkeit w durch die Effektivgeschwindigkeit (w/$\psi$) sowie der Rohrdurchmesser D durch den hydraulischen Kanaldurchmesser $d_H$ der Füllkörper oder Einbauten zu ersetzen, so dass gilt:

$$PD = Cw*\rho/2*(w/\psi)^2*1/d_H$$

mit

- $d_H$ [m] Hydraulischer Kanaldurchmesser
- $\psi$ [-] Leerrohranteil
- Cw [-] Widerstandsbeiwert des durchströmten Apparates mit Füllung

Die füllkörperspezifischen Daten $d_H$ und $\psi$ sind häufig Bestandteil der Lieferspezifikationen von Füllkörpern. Für eine Reihe von Füllkörpern sind Daten im oben erwähnten VDI-Wärmeatlas angegeben.

Der Leerrohranteil $\psi$ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F [$m^2/m^3$] der Füllkörper oder Einbauten (in der Regel bekannt oder experimentell bestimmbar) nach der einfachen Beziehung

$$dH = 4\psi/F.$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig n = 1, m = 0 (Ansatz nach S. Ergun, Chem. Engng. Progr. 48, (1948), 89), oder n = 1, m = 0,1 (Ansatz nach Brauer et al.) verwendet wird $K_1$, $K_2$ sind füllkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als

$$Re = w^*(\rho/\eta)^*D$$

für Leerrohre bzw.

$$Re = (w/\psi) * (\rho/\eta)^*dH$$

für Rohre mit Einbauten oder Füllkörpern. η [Pa*s] bezeichnet jeweils die Viskosität und ρ [$kg/m^3$] die Dichte des strömenden Mediums.

Der Druckverlust bei Zweiphasenströmungen (hier flüssig-flüssig für Aldehyd-Keton-Gemisch/Katalysatorlösung) steigt überproportional an. Meist wird nach Lockhart-Martinelli (in Brauer et al.) der Druckverlust der Zweiphasenströmung $P_{l1l2}$ auf den Druckverlust einer der beiden Phasen bezogen, zum Beispiel auf den Druckverlust der reinen strömenden flüssigen Phase $P_{l1}$, und in Beziehung zu dem Verhältnis des Druckverlustes der anderen allein strömend gedachten Phase $P_{l2}$ gesetzt.

Zur Berechnung von Druckverlusten in Zweiphasenströmungen werden häufig dimensionslose Drücke nach $\phi^2 = P_{l1l2}/P_{l1}$ und $X^2 = P_{l1}/P_{l2}$ eingesetzt. Der weitere Zusammenhang $\phi^2$ = Funktion($X^2$) ist vielfach untersucht. Beispiele finden sich in folgenden Literaturstellen:

Y. Sato, T.Hirose, F. Takahashi, M. Toda: "Pressure Loss and Liquid Hold Up in Packed Bed Reactor with Cocurrent Gas-Liquid Down Flow"; J. Chem. Chem. Eng. Of Japan, Vol 6 (Nr. 2), 1973, 147-152;

D. Sweeney: "A Correlation for Pressure Drop in Two-Phase Concurrent Flow in Packed Beds"; AIChE-Journal, Vol. 13, 7/1967, 663-669;

V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;

R P. Larkins, R P. White, D. W. Jeffrey: "Two-Phase Concurrent Flow in Packed Beds"; AIChE-Journal, Vol 7 (Nr. 2), 6/1961, 231-239 oder

N. Midoux, M. Favier, J.- C. Charpentier: " Flow Pattern, Pressure Loss and Liquid Holdup Data in Gas-Liquid Down-Flow Packed Beds with Foaming and Nonfoaming Liquids"; J. Chem. Eng. Of Japan, Vol 9 (Nr. 5), 1976, 350-356.

Häufig wird für die Berechnung der von Midoux vorgeschlagende Zusammenhang benutzt. Beispielsweise gilt

$$\Phi^2=1 + 1/X + 1,14\ X^{0,54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al.

Der Druckverlust der Zweiphasenströmung $P_{l1l2}$ ergibt sich aus dem experimentell bestimmten oder wie oben erläutert abgeschätzten Druckverlust der reinen strömenden Flüssigphase $P_{l1}$ dann mit

$$P_{l1l2} = \phi^2 * P_{l1}$$

Allgemein gilt mit der Reaktorlänge L [m]

$$PD = P_{l1l2}/L$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar; B sollte größer oder gleich 0,8, bevorzugt größer oder gleich 0,9 oder besonders bevorzugt größer oder gleich 1 sein.

Im Bereich B größer oder gleich 0,8 beginnt ein von oben nach unten betriebener Reaktor zu fluten. Es sei ausdrücklich darauf hingewiesen, dass bei Einhaltung dieser Bedingungen die Vorteile des erfindungsgemäßen Verfahrens auch dann erzielt werden, wenn der Reaktor von unten nach oben oder in anderer Richtung betrieben wird.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben.

Es ist somit ersichtlich, dass neben den Volumenströmen der einzelnen Phasen bzw. den hieraus abgeleiteten Leerrohrgeschwindigkeiten $w = V/(\pi D^2/4)$ die Apparateabmessungen des Reaktors (Länge L, Durchmesser D) sowie insbesondere die Daten der verwendeten Füllkörper (hydraulischer Durchmesser $d_H$, Leerrohranteil $\Psi$) eine wichtige Rolle spielen. Über die richtige Wahl dieser Parameter kann das Verfahren unschwer an die unterschiedlichsten Erfordernisse angepasst werden, wichtig ist nur die Einhaltung der Forderung B >= 0,8, bevorzugt B >= 0,9 und besonders bevorzugt B >= 1.

Bei einer langsamen Reaktion wird man beispielsweise den hydraulischen Durchmesser der Füllkörper klein bzw. ihre spezifische Oberfläche groß wählen, so dass die geforderten Bedingungen für B schon bei kleinen Strömungsgeschwindigkeiten erreicht werden. Man erhält auf diese Weise ausreichende Verweilzeiten über die Länge eines technisch vernünftig dimensionierten Reaktors. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der dispersen Phasen $M_2$. Im Fall der vorliegenden Erfindung ist der Massenstrom der Katalysatorphase $M_1$ wesentlich größer als der Massenstrom $M_2$ der dispersen Phase. Im erfindungsgemäßen Verfahren kann das Massenverhältnis $M_1/M_2$ der kontinuierlichen Phase ($M_1$) zu der dispersen Phasen ($M_2$) größer 2 sein, vorzugsweise gilt $M_1/M_2 > 10$. Strömungsverhältnisse mit $M_1/M_2 > 100$ sind durchaus möglich und häufig sogar vorteilhaft. Unter der Bedingung $M_1/M_2 > 2$ ist die Katalysatorphase die kontinuierliche Phase, während die disperse Phase. in feine Tropfen zerteilt wird.

Die Größe der feinen Tropfen können mit den üblichen ingenieurtechnischen Mitteln abgeschätzt werden. Es eignen sich hierfür Ansätze mit dimensionslosen Kennzahlen wie

$$d_S/d_H = k * Re_{l1l2}{}^m * We_{l1l2}{}^n$$

mit

| | |
|---|---|
| $d_S$ | Durchmesser der Tropfen nach Sauter (in Brauer et al.) |
| $d_H$ | hydraulischer Füllkörperdurchmesser, |
| $Re_{l1l2}$ | Reynoldszahl der Mehrphasenströmung = $w_{l1l2} * (\rho/\eta) * (dH/\Psi)$, |
| $We_{l1l2}$ | Weberzahl der Mehrphasenströmung = $w_{l1l2}{}^2 * (\rho/\sigma) * (dH/\Psi^2)$, |
| k, m, n | empirische Konstanten (bekannt oder durch Versuche zu ermitteln), |
| w | Leerrohrgeschwindigkeiten [m/s] = $V/(\pi D^2/4)$, |
| V | Volumenstrom unter Betriebsbedingungen [$m^3/s$], |
| $\rho$ | Dichte unter Betriebsbedingungen [$kg/m^3$], |
| $\eta$ | Viskosität unter Betriebsbedingungen [Pa*s] und |
| $\gamma$ | Grenzflächenspannung unter Betriebsbedingungen [N/m] |

und den Indices l1 (erste Flüssigphase) und l2 (zweite Flüssigphase).

Bei strukturierten Packungen wie Sulzer-SMV oder engen Rohren als Einbauten scheint es plausibel, dass ein berechneter Tropfendurchmesser $d_S$ größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H <= 1, \text{ vorzugsweise } < 0{,}9.$$

Aus dem berechneten Tropfendurchmesser lässt sich schliesslich eine Stoffübergangsfläche nach

$$A_s = 6\varphi_{12} d_s \; [m^2/m^3]$$

berechnen.

Für den Phasenanteil $\varphi_{l2}$der dispersen Phase (im Falle der Aldolkondensation ist die organische Phase dispergiert), kann mit den Leerrohrgeschwindigkeiten der Phasen

$$\varphi_{l2} \sim w_{l2}/w_{l1l2}$$

gesetzt werden.

Die Verweilzeit $\tau$ der den Reaktor durchströmenden Phasen lässt sich angenähert nach $\tau \sim L^*\psi / w_{l1l2}$. berechnen. Die Verweilzeit T beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise sehr geringer Anteil an Edukt an der Reaktionsmasse, dadurch bedingt wiederum sehr kurze Verweilzeit - bei vielen Mehrphasenreaktionen überraschend hohe Raum-Zeit-Ausbeuten erzielt. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zulässt.

Zweckmäßig wird das oder die Edukte durch die von der kontinuierlichen Phase in den Rohrreaktor eingebrachte Energie dispergiert.

Das erfindungsgemäße Verfahren kann sehr flexibel den unterschiedlichsten Anforderungen angepasst werden. Für spezielle Anforderungen bieten sich folgende Ausführungsformen des erfindungsgemäßen Verfahrens an:

Erfordert der Einsatzzweck eine sehr lange Durchmischungszone oder sind Ruhezonen z. B. zur Abnahme von Stoffströmen erforderlich, so bietet sich eine kaskadierende Anordnung von Rohrreaktoren mit Einbauten oder Füllkörpern an.

Eine Kaskadierung von Rohrreaktoren oder die alternative Anordnung von gepackten und leeren Rohrabschnitten ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, denkbar.

Auch die Wärmeabfuhr bei stark exothermen Reaktionen wie zum Beispiel bei der Aldolkondensation ist beim erfindungsgemäßen Verfahren unkritisch. Der hohe Durchsatz des Katalysatorkreislaufs wirkt als Wärmeträger, so dass selbst bei adiabatischer Fahrweise des Reaktors nur geringe Temperaturdifferenzen auftreten und eine homogene Temperaturverteilung im Reaktor ohne Temperaturspitzen resultiert. Die erzeugte Wärme lässt sich dann bequem durch einen beliebig im äußeren Katalysatorkreislauf angeordneten, konventionellen Wärmetauscher abführen oder zur Energiegewinnung ausnutzen. Zur besseren Wärmeabfuhr kann es unter Umständen günstig sein, den Katalysatorkreislauf noch höher zu fahren (also bei einem höheren B-Wert) als nach den Versuchsergebnissen notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig
- Der Katalysator wird geschont, die Desaktivierung ist sehr gering, eine kontinuierliche Ausschleusung wird minimiert.

Im Falle der Herstellung von α,β-ungesättigten Ketonen durch Aldolkondensation von einem Aldehyd mit einem Keton mit dem erfindungsgemäßen Verfahren kommt als weiterer Vorteil hinzu, dass aufgrund der hohen Reaktionsgeschwindigkeiten auch Aldehyde mit sehr geringer Löslichkeit in der Katalysatorphase wirtschaftlich zu den entsprechenden Aldolkondensationsprodukten umgesetzt werden können.

Als Lösungsmittel für die Herstellung der Katalysatorlösung bzw. -phase sind alle diejenigen Solventien geeignet, die folgende Bedingungen erfüllen:

- Das Lösungsmittel ist in der Produktphase wenig löslich
- Das Produkt löst sich nur wenig in der Katalysatorphase, die aus Katalysator und Lösungsmittel besteht.
- Das Lösungsmittel hat für den eingesetzten Katalysator eine genügend hohe Löslichkeit.

Ein bevorzugtes Lösungsmittel für den Katalysator ist Wasser oder ein homogenes Gemisch aus Wasser und einem organischen Lösungsmittel. Die Katalysatorphase kann auch mehr als ein Solvens enthalten, beispielsweise Wasser und Diethylenglykol.

Optional kann die Katalysatorphase Phasentransfer-, oberflächenaktive- oder amphiphile Reagenzien oder Tenside enthalten.

Als Katalysator können wasserlösliche, basische Verbindungen, z. B. Hydroxide, Hydogencarbonate, Carbonate oder Carboxylate in Form ihrer Alkali- oder Erdalkaliverbindungen verwendet werden. Vorzugsweise kommen Alkalilaugen zum Einsatz.
Die Konzentration des Katalysators in der Katalysatorlösung liegt zwischen 0,1 und 15 Massen-%, insbesondere zwischen 0,1 und 5 Massen-%.

Das erfindungsgemäße Verfahren ist für die Umsetzung von einem Aldehyd ( Struktur II) mit einem Keton (Struktur III), das eine Aldolkondensationreaktion eingehen kann, geeignet. Da bei dieser Reaktion der eingesetzte Aldehyd die reaktive Carbonyleinheit stellt, gibt es hinsichtlich der Struktur des Aldehydes keine Einschränkungen. Ketone stellen bei dieser Reaktion die Methylenkomponente. Es können daher nur Ketone, die zwei Wasserstoffatome am gleichen Atom in α-Stellung zur Carbonylgruppe haben, eingesetzt werden.

Beispielsweise sind die im Folgenden genannten Aldehyde für die erfindungsgemäße Umsetzung geeignet:

Formaldehyd, Acetaldehyd, Propanal, n-Butyraldehyd, Isobutyraldehyd, Crotonaldehyd, Valeraldehyd, 2-Methylbutanal, 3-Methylbutanal, Dimethylpropanal, Tiglinaldehyd, 3,3-Dimethylacrolein, n-Hexanal, Isohexanal, n-Heptanal, Citral, α- und β-Cyclocitral, Benzaldehyd, Zimtaldehyd, Phenylacetaldehyd, Hydrozimtaldehyd, 2-Phenylpropionaldehyd, Cyclohexylcarbaldehyd, Anisaldehyd, Farnesal, Phytal, Vitamin A-Aldehyd.

Weiterhin können Aldehyde oder Aldehydgemische, die durch Hydroformylierung von Olefinen oder Olefingemischen erzeugt worden sind, eingesetzt werden. Beispielsweise seien die $C_5$-Aldehydgemische, die bei der Hydroformylierung von $C_4$-Olefingemischen erhalten werden, oder die isomeren Isononanale, die bei der Hydroformylierung von technischen Dibutengemischen entstehen, genannt.

Darüber hinaus können ungesättigte Aldehyde, die durch Selbstkondensation eines Aldehyds, wie 2-Ethylhex-2-enal aus n-Butyraldehyd, entstanden sind, verwendet werden.

Als Ketone nach Formel III sind beispielsweise geeignet:
Aceton, Methylethylketon, Methyl-n-propylketon, Methyl-isopropylketon, Methyl-n-butylketon, Methyl-isobutylketon, Methyl-tert.-butylketon, Diethylketon, Diacetyl, 6-Methyl-5-hepten-2-on, 6-Methyl-3-hepten-2-on, Acetophenon, Cyclohexanon, Cyclohexylethylketon, Benzylmethylketon, Methylpropenylketon, Ethylpropenytketon, Mesityloxid, Propylpropenylketon, Isobutylidenaceton, 6-Methyl-3,5-heptadien-2-on, β-Jonon, Farnesylaceton, Geranylaceton, Cyclooctanon, Isophoron, 3,3,5-Trimethylhexanon, Cyclododecanon.

Sowohl der eingesetzte Aldehyd als auch das eingesetzte Keton kann unter Reaktionsbedingungen fest oder flüssig sein.(Formaldehyd wird als Lösung, bevorzugt in Wasser, eingesetzt.) Ist das Gemisch der Edukte fest oder wird ein festes Edukt allein in den Reaktor eingespeist, muss ein Lösemittel verwendet werden. Als Lösungsmittel werden unter Reaktionsbedingungen inerte, in der Katalysatorlösung kaum lösliche, Flüssigkeiten verwendet. Ebenso muss ein Lösungsmittel eingesetzt werden, wenn ein festes Produkt entsteht.

Darüber hinaus kann die Verwendung eines organischen Lösemittel, das in der Katalysatorphase nur wenig löslich ist, weitere Vorteile haben. Dieses Lösungsmittel kann zusätzlich in der Produktphase gut löslich sein. Beispielsweise kann der Zusatz eines Lösemittels die Selektivität für die Bildung des Zielproduktes erhöhen. Weiterhin kann die Verwendung eines Lösungsmittel die Reaktionsführung und die Aufarbeitung des Reaktionsgemisches erleichtern, wie noch beschrieben wird. Es ist auch der umgekehrte Fall denkbar, nämlich der Einsatz eines Lösungsmittels, das in der Produktphase nicht gut aber in der Katalysatorphase gut löslich ist.
Geeignete Lösemittel können beispielsweise Ether oder Kohlenwasserstoffe sein. Ein bevorzugtes Lösemittel ist Cyclohexan.

Die beiden Edukte können getrennt oder als Gemisch in den Reaktor eingespeist werden.

Das molare Verhältnis zwischen Aldehyd und Keton liegt im Bereich von 2/1 bis 1/10, bevorzugt im Bereich von 1/1 bis 1/3.

Die spezifische Verweilzeit und der Belastungsfaktor sind entsprechend des erfindungsgemäßen Verfahrens zu wählen. Hierzu kann in einem oder mehreren Rohrreaktor(en) mit Einbauten entsprechend der vorangegangenen Beschreibung gearbeitet werden.

Die erfindungsgemäße Umsetzung kann in einem Temperaturbereich von 30°C bis 200°C, vorzugsweise im Bereich 60°C bis 150°C erfolgen; hierbei liegt der Gesamtdruck zwischen 0,1 bar und 25 bar.

Das Reaktionsrohr kann im Gleichstrom von oben nach unten oder umgekehrt durchströmt werden. Aus Sicherheitsgründen wird der Beschickung von oben der Vorzug gegeben.

Die Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen.

Die abgeführte Reaktionswärme kann so sehr einfach genutzt werden, z.B. im Prozess selbst, zur Beheizung einer Destillationseinrichtung oder zur Erzeugung von Dampf.

Das den Reaktor verlassende Flüssigkeitsgemisch wird in einem Flüssig-Flüssig-Trennbehälter in die Katalysatorphase und in die Produktphase mechanisch getrennt. Dies kann in Absitzbehältern verschiedener Bauart oder Zentrifugen erfolgen. Aus Kostengründen werden Absitzbehälter bevorzugt.

Die Verweilzeiten in der Trennvorrichtung sind zwar nicht grundsätzlich kritisch, werden aber vorteilhaft klein gehalten. Dies hat folgende Vorteile: Die Trennvorrichtung ist klein und das Investment dafür entsprechend gering. Bei kurzen Verweilzeiten treten praktisch keine Nebenreaktionen im Trennbehälter auf. Damit die Trennung der Phasen schnell erfolgt, muss der Dichteunterschied der beiden Phasen genügend groß und müssen ihre Viskositäten gering sein. Alle drei Größen sind eine Funktion der Temperatur und können durch orientierende Versuche leicht ermittelt werden.

Darüber hinaus kann die Dichte und Viskosität der Katalysatorlösung durch Auswahl des Solvens und der Katalysatorkonzentration variiert werden. Als weitere Möglichkeit kann die Dichte und Viskosität der Produktphase durch Zusatz eines Lösemittels verändert werden. Die Phasentrennung kann in einem weiten Temperaturbereich vorgenommen werden. Dabei kann die Trenntemperatur auch höher sein als die Temperatur des Reaktionsaustrags am Reaktorausgang. Aus energetischen Gründen ist es jedoch nicht günstig, eine höhere Temperatur als die Flüssigkeitstemperatur am Reaktorausgang anzuwenden. Als tiefstmögliche Temperatur ist der Stockpunkt einer der beiden flüssigen Phasen anzusehen. Im Hinblick auf kurze Trennzeiten werden jedoch, wie oben erwähnt, keine zu tiefen Temperaturen gewählt.

Das entstehende Reaktionswasser muss aus dem Reaktionssystem entfernt werden. Wird mit der organischen Produktphase mehr Wasser abgetrennt, als Reaktionswasser entsteht, muss die Wasserfehlmenge ständig nachdosiert werden. Einfacher ist die Reaktionsführung, wenn gerade so viel Wasser mit der organischen Produktphase abgezogen würde, wie dem Reaktionswasser entspricht. Die Löslichkeit des Wassers in der Produktphase und die dadurch bedingte Wassermenge darin kann durch Zusatz eines Lösemittels eingestellt werden, sodass die Verwendung eines Lösemittels zweckmäßig sein kann.

Wenn dagegen die Katalysatorlösung durch das Reaktionswasser verdünnt wird, muss ein Teil des Wassers daraus entfernt werden. Dies kann durch Abdestillieren von Wasser aus der Katalysatorlösung geschehen. Optional kann das Reaktionsgemisch vor der Phasentrennung geflasht werden, wodurch eine Aufkonzentrierung der Katalysatorlösung erfolgen kann.

Aus der abgetrennten organischen Produktphase kann Wasser destillativ abgetrennt werden. Dies ist dann besonders einfach, wenn Wasser mit einer Komponente der Produktphase, die in ausreichender Menge vorliegt, ein Minimum-Hetero-Azeotrop bilden kann. Dieser Fall kann, unabhängig von den Reaktionspartnern, durch Zusatz eines entsprechenden mit Wasser ein Minimum-Hetero-Azeotrop bildendes Lösungsmittel, wie beispielsweise Cyclohexan, erreicht werden.

Der Produktstrom wird nach bekannten Verfahren, z.B. durch Destillation, in Produkt, Edukt, Nebenprodukt und gegebenenfalls Lösemittel aufgetrennt. Die abgetrennten Edukte und gegebenenfalls das Lösemittel werden in den Prozess zurückgeführt. Ebenso ist es zweckmäßig, einen Teil der Nebenprodukte, nämlich die Additionsprodukte zurückzuführen, wodurch die Selektivität der Umsetzung erhöht werden kann.

Die abgetrennte Katalysatorlösung wird, ggf. nach Ausschleusung einer kleinen Teilmenge und entsprechendem Ersatz durch frische Katalysatorlösung, in den Reaktor zurückgeführt.

Die im erfindungsgemäßen Verfahren erzeugten $\alpha,\beta$-ungesättigten Ketone können zu den entsprechenden gesättigten Ketonen hydriert werden.

Die nach dem erfindungsgemäßen Verfahren hergestellten Verbindungen sind wertvolle Zwischenprodukte für die Herstellung von Riechstoffen, Lösungsmitteln, Farbstoffen, Kunststoffen und Pharmazeutika. So ist beispielsweise 6-Methythept-3-en-2-on ein wichtiges Zwischenprodukt bei der Synthese von Vitamin E. Ein Teil dieser Stoffe wird selbst als Riechstoff verwendet. Weiterhin können die ungesättigten Ketone zu den gesättigten Alkoholen hydriert werden, die beispielsweise zur Herstellung von Estern oder Olefinen verwendet werden können.

Die folgenden Beispiele sollen die Erfindung beschreiben, ohne deren Anwendungsbreite einzuschränken, die aus den Patentansprüchen hervorgeht.

Die den Beispielen beigefügte erste Tabelle beschreibt zunächst die Katalysatorzusammensetzung in Massenprozenten, dann die Menge des Eduktes und dessen Zusammensetzung in Massenprozenten der gaschromatographischen Analyse.

Im unteren Bereich der jeweils zweiten Tabelle ist die Produktzusammensetzung ebenfalls in Massenprozenten der gaschromatographischen Analyse aufgelistet.

Im oberen Bereich der zweiten Tabelle sind die Raum-Zeit-Ausbeute (RZA), der Umsatz (U) der Aldehyde, die Selektivität (S) zu den gewünschten Aldolkondensationsprodukten und der Belastungsfaktor (B) (außer Beispiel 1) angegeben. Bei der beschriebenen Katalysatorzusammensetzung ist zu beachten, dass es sich bei den Beispielen um Startwerte handelt. Der Anteil an NaOH wurde durch das Reaktionswasser der Aldolkondensation leicht verdünnt. Darüber hinaus führt die parallel zu Aldolkondensation ablaufende Cannizzaro-Reaktion zur Neutralisation des alkalischen Katalysators. Beide Effekte sind im beobachteten Zeitraum aber so gering, dass dies für die Beschreibung der Versuche und der Versuchsergebnisse unwesentlich ist.

Beispiel 1 (Vergleichsbeispiel):

Dieses Beispiel beschreibt ein Verfahren für die Aldolkondensation von Aceton (Ac) und 3-Methylbutanal (3-MBA) zu 6-Methyl-3-hepten-2-on (6-MH). In diesem Vergleichsbeispiel wurde die herkömmliche Rührkesseltechnologie angewendet. Die Bildung der Nebenprodukte 4-Methyl-3-penten-2-on (4-MP), 3-Methyl-2-isopropyl-2-butenal (3-MiPB), 5-Methyl-2-isopropyl-2-hexenal (5-MiPH), 4-Hydroxy-6-Methylheptan-2-on (6-HMH) sowie den sonstigen Hochsiedern (HS) sind in der nachfolgenden Tabelle in Gew.-% angegeben.

Im Rührkesselreaktor wurden 1000 g Katalysator vorgelegt. Hierzu wurde die Eduktmischung gegeben. Die Reaktion wurde bei einer Temperatur von 80°C bei Eigendruck der Reaktionsteilnehmer durchgeführt.

| | |
|---|---|
| Katalysator [g] | 1000 |
| c NaOH [Gew.-%] | 7 |
| Wasser [Gew.-%] | 89 |
| Aceton [Gew.-%] | 4 |
| | |
| Edukt [l/h] | 424 |
| Ac [Gew-%] | 28,6 |
| 3-MBA [Gew-%] | 38,2 |
| CH [Gew-%] | 33,2 |

Folgendes Ergebnis wurde erzielt:

| | |
|---|---|
| RZA [t/m$^3$/h] | 1,4 |
| U | 0,75 |
| S | 0,67 |
| | |
| Ac | 23,61 |
| 3-MBA | 10,10 |
| 6-MH | 44,37 |
| 4-MP | 0,16 |
| 3-MiPB | 0,27 |

(fortgesetzt)

| | |
|---|---|
| 5-MiPH | 0,72 |
| 6-HMH | 17,38 |
| HS | 3,39 |

Zu Beispiel 2 und 3

Die Aldolisierung erfolgte in einer Versuchsapparatur, die schematisch in Fig. 1 dargestellt ist. Hierin wird mit einer Pumpe 1 die kontinuierliche Katalysatorphase 2 im Kreislauf gepumpt. Zum Katalysator wird der Aldehyd oder die Aldehydmischung durch Leitung 3 oder verschiedene Aldehyde getrennt durch die Leitungen 3 und 4 zugemischt. Für die nachfolgend aufgeführten Beispiele 2 und 3 wurden die Edukte ausschliesslich über Leitung 3 zugemischt. Die Mehrphasenmischung 5 wird bei den Beispielen 3 bis 14 durch den Rohrreaktor 6 mit einer Länge von 3 m und einem Durchmesser von 17,3 mm gepumpt, der mit statischen Mischelementen mit einem hydraulischen Durchmesser von 2 mm versehen war. Die resultierende Mischung 7, bestehend aus dem Reaktionsprodukt, nicht umgesetztem Edukt und dem Katalysator können im Gasabscheider 8 von leicht flüchtigen Bestandteilen durch Ausschleusung in Leitung 9 befreit werden. Für die nachfolgend aufgeführten Beispiele, außer 2, war diese Leitung geschlossen.

Der nach der Entgasung 8 anfallende Flüssigkeitsstrom 10 wird in einen Phasentrennbehälter 11 geleitet. Hier wird die wässrige Katalysatorphase 2 abgetrennt und erneut dem Kreislauf zugeführt. Die über ein Wehr gelaufene organische Phase, die das Reaktionsprodukt enthält, wird aus Leitung 12 entnommen.

Die Reaktionswärme kann über außerhalb des Reaktors liegende Wärmetauscher 13, 14 und 15 abgeführt werden.

Beispiel 2:

Dieses Beispiel beschreibt das erfindungsgemäße Verfahren für die Aldolkondensation von Aceton (Ac) und 3-Methylbutanal (3-MBA) zu 6-Methyl-3-hepten-2-on (6-MH). Die Bildung der Nebenprodukte 4-Methyl-3-penten-2-on (4-MP), 3-Methyl-2-isopropyl-2-butenal (3-MiPB), 5-Methyl-2-isopropyl-2-hexenal (5-MiPH), 4-Hydroxy-6-Methylheptan-2-on (6-HMH) sowie den sonstigen Hochsiedern (HS) sind in der nachfolgenden Tabelle in Gew.-% angegeben.

Der Reaktor wurde mit einer Katalysatorbelastung von 400 kg/h bei einer Temperatur von 80 °C bei Eigendruck der Reaktionsteilnehmer durchströmt.

| | |
|---|---|
| Katalysator [kg] | 4,5 |
| c NaOH [%] | 6,7 |
| Wasser [%] | 89,2 |
| Aceton | 4,1 |
| | |
| Edukt [l/h] | 5,24 |
| Ac [Gew-%] | 42,36 |
| 3-MBA [Gew-%] | 33,34 |
| CH [Gew-%] | 24,30 |

Folgendes Ergebnis wurde erzielt:

| | |
|---|---|
| RZA [t/m$^3$/h] | 3,2 |
| U | 0,86 |
| S | 0,95 |
| B | 15,34 |
| | |
| Ac | 33,27 |

(fortgesetzt)

| | |
|---|---|
| 3-MBA | 5,26 |
| 6-MH | 58,37 |
| 4-MP | 0,66 |
| 3-MiPB | 0,42 |
| 5-MiPH | 0,3 |
| 6-HMH | 0,5 |
| HS | 1,2 |

Es wird deutlich, dass mit dem erfindungsgemäßen Verfahren deutlich höhere Selektivitäten bei größeren Raum-Zeit-Ausbeuten erzielt werden.

Beispiel 3:

Dieses Beispiel beschreibt das erfindungsgemäße Verfahren für die Aldolkondensation von Aceton (Ac) und Pentanal (PAL) zu 3-Octen-2-on (3-ON). Die Bildung der Nebenprodukte 4-Methyl-3-penten-2-on (4-MP), 4-Hydroxy-4-methyl-3-pentan-2-on (4-HMP), 4-Hydroxy-3-octen-2-on (4-HON), 2-Propyl-2-heptenal (2-PHL) sowie den sonstigen Hochsiedern (HS) sind in der nachfolgenden Tabelle in Gew.-% angegeben.

Der Reaktor wurde mit einer Katalysatorbelastung von 400 kg/h bei einer Temperatur von 80°C bei Eigendruck der Reaktionsteilnehmer durchströmt.

| | |
|---|---|
| Katalysator [g] | 4981 |
| c NaOH [%] | 4,0 |
| Wasser [%] | 91,8 |
| Aceton | 4,2 |
| | |
| Edukt [l/h] | 4,28 |
| Ac [Gew-%] | 48,04 |
| 3-MBA [Gew-%] | 51,96 |

Folgendes Ergebnis wurde erzielt:

| | |
|---|---|
| RZA [t/m$^3$/h] | 2,1 |
| U | 0,95 |
| S | 64,00 |
| B | 14,72 |
| | |
| Ac | 22,52 |
| PAL | 3,04 |
| 4-MP | 0,35 |
| 4-HMP | 0,23 |
| 3-ON | 47,46 |
| 4-HON | 8,03 |
| 2-PHL | 9,49 |
| HS | 8,88 |

## Patentansprüche

1. Verfahren zur Herstellung von α,β-ungesättigten Ketonen der allgemeinen Struktur I

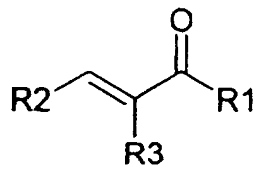

I

in der R1 und R2 für einen verzweigten oder unverzweigten, gesättigten oder ungesättigten aliphatischen oder cycloaliphatisch-aliphatischen Kohlenwasserstoffrest mit 1 bis 20, vorzugsweise 1-16 C-Atomen oder einen gesättigten oder ungesättigten cycloaliphatischen Kohlenwasserstoffrest mit 5 bis 12 C-Atomen, der auch Alkylgruppen als Substituenten und/oder eine Endoalkylengruppe enthalten kann oder aber auch für einen araliphatischen Kohlenwasserstoffrest mit 7 bis 15 C-Atomen, vorzugsweise eine Benzylgruppe, oder einen aromatischen Kohlenwasserstoffrest, vorzugsweise eine Phenylgruppe stehen, R3 Wasserstoff oder einen aliphatischen Kohlenwasserstoffrest mit 1 bis 10 C-Atomen bedeutet, und darüber hinaus R1 und R3 auch zusammen mit den beiden benachbarten Kohlenstoffatomen Glieder eines gemeinsamen alicyclischen Ringes bezeichnen können und weiterhin eine oder mehrere Methylengruppen, die nicht in α-Stellung zu einer Carbonylgruppe stehen, in den Resten R1,R2 und R3 durch ein Sauerstoff- oder Schwefelatom substituiert sein können, durch Umsetzung von einem Aldehyd der allgemeinen Struktur II mit einem Keton der allgemeinen Struktur III, in der R1, R2 und R3 die vorgenannten Bedeutung haben, in flüssiger Phase in einem Rohrreaktor,

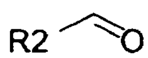

II

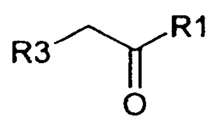

III

**dadurch gekennzeichnet,**
**dass** der Katalysator in der kontinuierlichen Phase und mindestens ein Edukt in einer dispergierten Phase enthalten ist und das Massenverhältnis der kontinuierlichen Phase zu der dispergierten Phase größer 2 ist und der Belastungsfaktor des Rohrreaktors gleich oder größer 0,8 ist.

2. Verfahren nach Anspruch 1,
**dadurch gekennzeichnet,**
**dass** Aldehyde mit 1 bis 15 C-Atomen eingesetzt werden.

3. Verfahren nach den Ansprüchen 1 und 2,
**dadurch gekennzeichnet,**
**dass** Aldehyde mit 4 oder 5-C-Atomen verwendet werden.

4. Verfahren nach einem der Ansprüche 1 bis 3,
**dadurch gekennzeichnet,**
**dass** Ketone mit 3 bis 15 C-Atomen verwendet werden.

5. Verfahren nach Anspruch 4,
**dadurch gekennzeichnet,**
**dass** als Keton Aceton eingesetzt wird.

**6.** Verfahren nach einem der Ansprüche 1 bis 5,
**dadurch gekennzeichnet,**
**dass** als Katalysator eine wasserlösliche basische Verbindung verwendet wird.

**7.** Verfahren nach Anspruch 6,
**dadurch gekennzeichnet,**
**dass** als Katalysator Hydoxide, Hydrogencarbonate, Carbonate, Carboxylate oder ihre Gemische in Form ihrer Alkali- oder Erdalkaliverbindungen verwendet werden, insbesondere Alkalilaugen.

**8.** Verfahren nach Anspruch 7,
**dadurch gekennzeichnet,**
**dass** der Katalysator in Konzentrationen von 0,1 bis 15 Massen-% in der kontinuierlichen Phase vorliegt, insbesondere in Konzentrationen von 0,1 bis 5 Massen-%.

**9.** Verfahren nach einem der Ansprüche 1 bis 8;
**dadurch gekennzeichnet,**
**dass** als Solvens für den Katalysator Wasser oder ein homogenes Gemisch aus Wasser und einem organischen Lösungsmittel eingesetzt wird.

**10.** Verfahren nach Anspruch 9,
**dadurch gekennzeichnet,**
**dass** Wasser als Lösungsmittel für die kontinuierliche Katalysatorphase verwendet wird.

**11.** Verfahren nach Anspruch 9,
**dadurch gekennzeichnet,**
**dass** ein Gemisch aus Wasser und Ethylenglykol als Solvens für die Katalysatorphase verwendet wird.

**12.** Verfahren nach einem der Ansprüche 1 bis 11,
**dadurch gekennzeichnet,**
**dass** der Belastungsfaktor B größer oder gleich 0,9 ist.

**13.** Verfahren nach einem der Ansprüche 1 bis 11,
**dadurch gekennzeichnet,**
**dass** der Belastungsfaktor B größer oder gleich 1,0 ist.

**14.** Verfahren nach einem der Ansprüche 1 bis 13,
**dadurch gekennzeichnet,**
**dass** das Edukt durch die von der kontinuierlichen Phase in den Rohrreaktor eingebrachte Energie dispergiert wird.

**15.** Verfahren nach einem der Ansprüche 1 bis 14,
**dadurch gekennzeichnet,**
**dass** Aldehyd und Keton im molaren Verhältnis von 2/1 bis 1/10, insbesondere im molaren Verhältnis 1/1 bis 1/3 eingesetzt werden.

**16.** Verfahren nach einem der Ansprüche 1 bis 15,
**dadurch gekennzeichnet,**
**dass** ein Lösemittel eingesetzt wird, das mit Wasser ein Minimum-Hetero-Azeotrop bildet, eingesetzt wird.

**17.** Verfahren nach Anspruch 16,
**dadurch gekennzeichnet,**
**dass** Cyclohexan als Lösemittel eingesetzt wird.

**18.** Verfahren nach einem der Ansprüche 1 bis 17,
**dadurch gekennzeichnet**
**dass** die erzeugten $\alpha,\beta$-ungesättigten Ketone zu den entsprechenden gesättigten Ketonen hydriert werden.

**19.** Verfahren nach einem der Ansprüche 1 bis 17,
**dadurch gekennzeichnet,**

**dass** die erzeugten α,β-ungesättigten Ketone zu den entsprechenden gesättigten Alkoholen hydriert werden.

## Claims

1. A process for preparing an α,β-unsaturated ketone of the general structure I

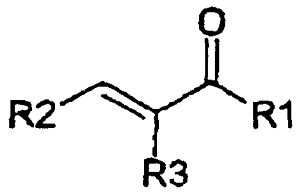

I

where R1 and R2 each represent a branched or unbranched, saturated or unsaturated aliphatic or cycloaliphatic-aliphatic hydrocarbon radical of 1 to 20, preferably 1 to 16, carbon atoms or a saturated or unsaturated cycloaliphatic hydrocarbon radical of 5 to 12 carbon atoms which may contain alkyl groups as substituents and/or an endoalkylene group or else each represent an araliphatic hydrocarbon radical of 7 to 15 carbon atoms, preferably a benzyl group, or an aromatic hydrocarbon radical, preferably a phenyl group, R3 represents hydrogen or an aliphatic hydrocarbon radical of 1 to 10 carbon atoms, or else R1 and R3 combine with the two adjacent carbon atoms to form members of a common alicyclic ring and, furthermore, in R1, R2 and R3, one or more methylene groups which are not α-disposed relative to a carbonyl group may be substituted by an oxygen or sulfur atom, by reacting an aldehyde of the general structure II with a ketone of the general structure III, where R1, R2 and R3 are each as defined above, in the liquid phase in a tubular reactor

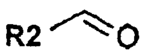

II

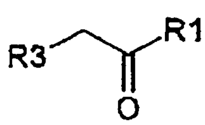

III

**characterized in that** the catalyst is present in the continuous phase and at least one reactant is present in a dispersed phase the mass ratio of continuous phase to dispersed phase is greater than 2 and the loading factor of the tubular reactor is not less than 0.8.

2. A process according to claim 1, **characterized in that** an aldehyde of 1 to 15 carbon atoms is used.

3. A process according to either of claims 1 and 2, **characterized in that** an aldehyde of 4 or 5 carbon atoms is used.

4. A process according to any one of claims 1 to 3, **characterized in that** a ketone of 3 to 15 carbon atoms is used.

5. A process according to claim 4, **characterized in that** acetone is used as ketone.

6. A process according to any one of claims 1 to 5, **characterized in that** the catalyst used is a water-soluble basic compound.

7. A process according to claim 6, **characterized in that** the catalyst used is selected from hydroxides, bicarbonates, carbonates, carboxylates or their mixtures in the form of their alkali or alkaline earth metal compounds, especially aqueous alkali metal hydroxide solutions.

8. A process according to claim 7, **characterized in that** the catalyst is present in the continuous phase in a concentration in the range from 0.1 to 15 mass%, especially in a concentration in the range from 0.1 to 5 mass%.

9. A process according to any one of claims 1 to 8, **characterized in that** water or a homogeneous mixture of water and an organic solvent is used as solvent for the catalyst.

10. A process according to claim 9, **characterized in that** water is used as solvent for the continuous catalyst phase.

11. A process according to claim 9, **characterized in that** a mixture of water and ethylene glycol is used as solvent for the catalyst phase.

12. A process according to any one of claims 1 to 11, **characterized in that** the loading factor B is not less than 0.9.

13. A process according to any one of claims 1 to 11, **characterized in that** the loading factor B is not less than 1.0.

14. A process according to any one of claims 1 to 13, **characterized in that** the reactant is dispersed by the energy introduced into the tubular reactor by the continuous phase.

15. A process according to any one of claims 1 to 14, **characterized in that** aldehyde and ketone are used in a molar ratio in the range from 2/1 to 1/10, especially in a molar ratio in the range from 1/1 to 1/3.

16. A process according to any one of claims 1 to 15, **characterized in that** a solvent is used which forms a minimum heteroazeotrope with water.

17. A process according to claim 16, **characterized in that** cyclohexane is used as solvent.

18. A process according to any one of claims 1 to 17, **characterized in that** the α,β-unsaturated ketone produced is hydrogenated to form the corresponding saturated ketone.

19. A process according to any one of claims 1 to 17, **characterized in that** the α,β-unsaturated ketone produced is hydrogenated to form the corresponding saturated alcohol.

**Revendications**

1. Procédé de préparation de cétones α-β-insaturées de structure générale I

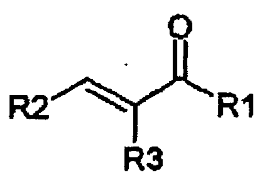

I

dans laquelle R1 et R2 représentent un radical hydrocarbure aliphatique ou cycloaliphatique-aliphatique ramifié ou non ramifié, saturé ou insaturé, portant 1 à 20 atomes, de préférence 1 à 16 atomes de C ou bien un radical hydrocarbure cycloaliphatique saturé ou insaturé portant 5 à 12 atomes de carbone qui peut aussi renfermer des groupes alkyle en tant que substituants et/ou un groupe endoalkylène ou bien aussi un radical hydrocarbure araliphatique portant 7 à 15 atomes de C, de préférence un groupe benzyle ou un radical hydrocarbure aromatique, de préférence, un groupe phényle, R3 signifie un atome d'hydrogène ou un radical hydrocarbure aliphatique portant 1 à 10 atomes de C, et, de plus à partir de là R1 et R3 peuvent ensemble aussi former, avec les deux atomes de carbone voisins, des chaînons d'un cycle alicylique commun et en outre un ou plusieurs groupes méthylène, qui ne sont pas en position α par rapport à un groupe carbonyle, peuvent être substitués par un atome d'oxygène ou de soufre dans les radicaux R1, R2 et R3, par conversion d'un aldéhyde de structure générale II avec une cétone de structure générale III, dans laquelle R1, R2 et R3 ont la signification précitée, dans la phase liquide dans un

réacteur tubulaire,

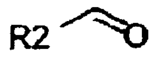

II

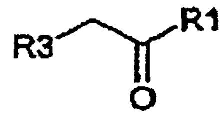

III

**caractérisé en ce que**
le catalyseur se trouve dans la phase continue et au moins un éduit est contenu dans une phase dispersée, le rapport molaire de la phase continue par rapport à la phase dispersée est supérieur à 2 et le facteur de charge du réacteur tubulaire est supérieur ou égal à 0,8.

**2.** Procédé selon la revendication 1,
**caractérisé en ce qu'**
on utilise des aldéhydes portant 1 à 15 atomes de C.

**3.** Procédé selon les revendications 1 et 2,
**caractérisé en ce qu'**
on utilise des aldéhydes portant 4 ou 5 atomes de C.

**4.** Procédé selon l'une quelconque des revendications 1 à 3,
**caractérisé en ce qu'**
on utilise des cétones portant 3 à 15 atomes de C.

**5.** Procédé selon la revendication 4,
**caractérisé en ce qu'**
on utilise de l'acétone comme cétone.

**6.** Procédé selon l'une quelconque des revendications 1 à 5,
**caractérisé en ce qu'**
on utilise un composé basique hydrosoluble en tant que catalyseur.

**7.** Procédé selon la revendication 6,
**caractérisé en ce que**
comme catalyseur, on utilise des hydroxydes, des carbonates d'hydrogène, des carbonates, des carboxylates ou leurs mélanges sous forme de leurs composés alcalins ou alcalino-terreux, en particulier des lessives alcalines.

**8.** Procédé selon la revendication 7,
**caractérisé en ce que**
le catalyseur se présente dans des concentrations de 0, 1 à 15% en masse dans la phase continue, en particulier dans des concentrations de 0,1 à 5 % en masse.

**9.** Procédé selon l'une quelconque des revendications 1 à 8,
**caractérisé en ce que**
comme solvant pour le catalyseur, on utilise de l'eau ou un mélange homogène d'eau et d'un solvant organique.

**10.** Procédé selon la revendication 9,
**caractérisé en ce qu'**
on utilise de l'eau en tant que solvant pour la phase de catalyse continue.

**11.** Procédé selon la revendication 9,
**caractérisé en ce qu'**
on utilise un mélange d'eau et d'éthylène glycol en tant que solvant pour la phase de catalyse.

**12.** Procédé selon l'une quelconque des revendications 1 à 11,

**caractérisé en ce que**
le facteur de charge B est supérieur ou égal à 0,9.

**13.** Procédé selon l'une quelconque des revendications 1 à 11,
**caractérisé en ce que**
le rapport de charge B est supérieur ou égal à 1,0.

**14.** Procédé selon l'une quelconque des revendications 1 à 13,
**caractérisé en ce que**
l'éduit est dispersé par l'énergie introduite par la phase continue dans le réacteur tubulaire.

**15.** Procédé selon l'une quelconque des revendications 1 à 14,
**caractérisé en ce qu'**
on utilise un aldéhyde et une cétone dans un rapport molaire de 2/1 à 1/10, en particulier dans un rapport molaire de 1/1 à 1/3.

**16.** Procédé selon l'une quelconque des revendications 1 à 15,
**caractérisé en ce qu'**
on utilise un solvant qui forme un hétéro-azéotrope minimum avec l'eau.

**17.** Procédé selon la revendication 16,
**caractérisé en ce qu'**
on utilise du cyclohexane en tant que solvant.

**18.** Procédé selon l'une quelconque des revendications 1 à 17,
**caractérisé en ce que**
les cétones $\alpha$-$\beta$-insaturées produites sont hydrogénées en cétones saturées correspondantes.

**19.** Procédé selon l'une quelconque des revendications 1 à 17,
**caractérisé en ce que**
les cétones $\alpha$-$\beta$-insaturées produites sont hydrogénées en alcools saturés correspondants.

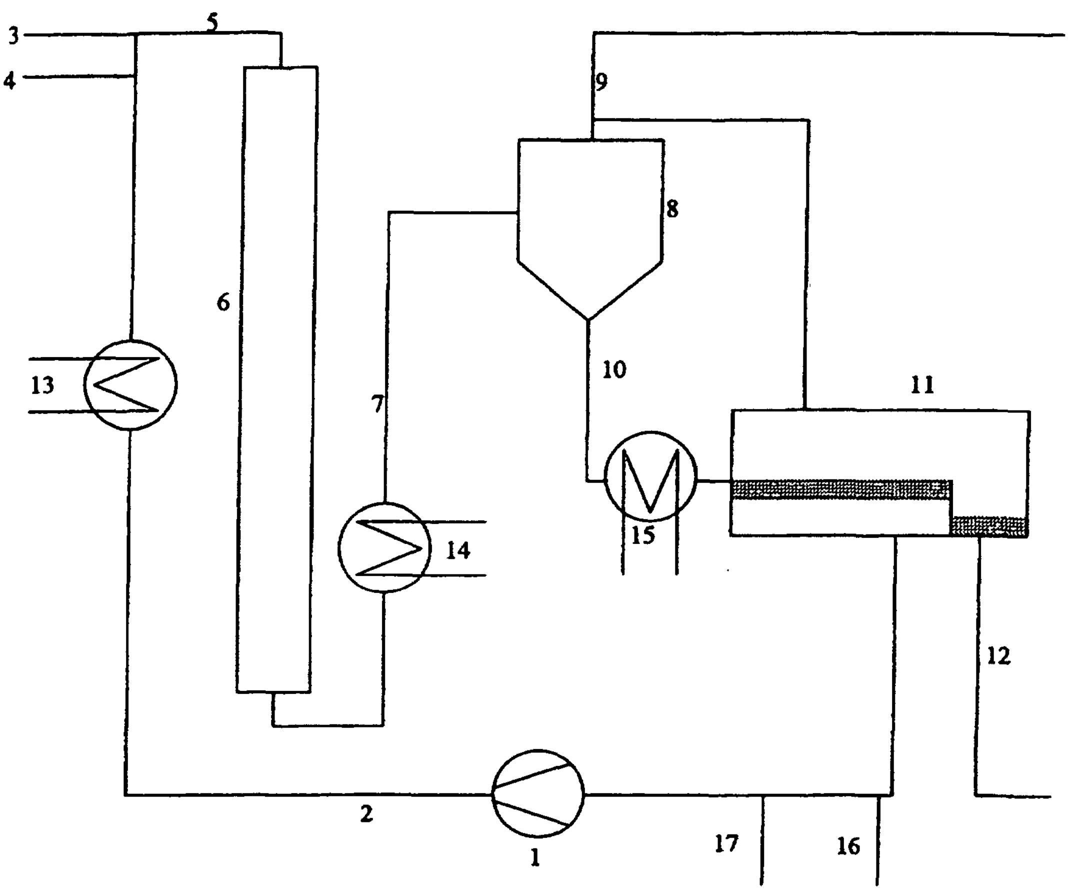


Figur 1